(19) Europäisches Patentamt
European Patent Office
Office européen des brevets

(11) Veröffentlichungsnummer : **0 182 054 B1**

# (12) EUROPÄISCHE PATENTSCHRIFT

(45) Veröffentlichungstag der Patentschrift : **07.03.90**

(51) Int. Cl.[5] : **C 07 D319/06**, C 07 D339/08, C 09 K 19/00

(21) Anmeldenummer : **85112492.5**

(22) Anmeldetag : **03.10.85**

## (54) Heterocyclische Verbindungen.

(30) Priorität : **17.10.84 DE 3437935**

(43) Veröffentlichungstag der Anmeldung : **28.05.86 Patentblatt 86/22**

(45) Bekanntmachung des Hinweises auf die Patenterteilung : **07.03.90 Patentblatt 90/10**

(84) Benannte Vertragsstaaten : **CH DE FR GB LI**

(56) Entgegenhaltungen :
**DE-A- 2 519 161**
**GB-A- 2 044 767**
**GB-A- 2 046 742**
**GB-A- 2 073 175**
**US-A- 3 676 500**
**MOLECULAR CRYSTALS AND LIQUID CRYSTALS + LETTERS, Band 123, Nr. 1/4, 1985, Seiten 137-141, Gordon and Breach, New York, US; H.-M. VORBRODT et al.: "New liquid crystalline 2,5-disubstituted 1,3-dithians and 1,3-dioxans"**

(73) Patentinhaber : **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder : **Krause, Joachim, Dr.**
**Samuel-Morse-Strasse 14**
**D-6110 Dieburg (DE)**
Erfinder : **Fuss, Peter, Dr.**
**Im Seegraben 5**
**D-6109 Mühltal-Traisa (DE)**
Erfinder : **Hittich, Reinhard, Dr.**
**Am Kirchberg 11**
**D-6101 Modautal 1 (DE)**
Erfinder : **Scheuble, Bernhard, Dr.**
**Am Grenzweg 18**
**D-6146 Alsbach (DE)**

**EP 0 182 054 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Verbindungen der Formel I

$$R^1\text{-}A^1\text{-}Z^1\text{-}A^2\text{-}R^2 \qquad \text{I}$$

worin

$R^1$ und $R^2$ jeweils eine Alkylgruppe mit 1-10 C-Atomen, worin auch eine oder zwei nicht benachbarte $CH_2$-Gruppen durch O-Atome und/oder CO-Gruppen und/oder —OCO-Gruppen und/oder —COO-Gruppen und/oder —CH=CH-Gruppen ersetzt sein können, F, Cl, Br, CN oder $R^3\text{-}A^3\text{-}Z^2$-,

$A^1$ -A-, -$A^4$-A- oder -A-$A^4$-,

A eine Gruppe ausgewählt aus den Formeln (B) und (D)

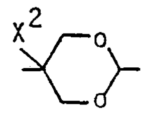
(B)

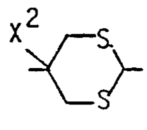
(D)

worin $X^2$ F, Cl oder CN bedeutet,

$A^2$, $A^3$ und $A^4$ jeweils unsubstituierte oder durch ein oder zwei F- und/oder Cl-Atome und/oder $CH_3$-Gruppen und/oder CN-Gruppen substituierte 1,4-Phenylen-Gruppen, worin auch eine oder zwei CH-Gruppen durch N-Atome ersetzt sein können, 1,4-Cyclohexylen-Gruppen, worin auch eine oder zwei nicht benachbarte $CH_2$-Gruppen durch O-Atome ersetzt sein können, 1,3-Dithian-2,5-diyl-, Piperidin-1,4-diyl-, 1,4-Bicyclo [2, 2, 2]-octylen-, Decahydronaphthalin-2,6-diyl- oder 1,2,3,4-Tetrahydronaphthalin-2,6-diyl-Gruppen,

$Z^1$ und $Z^2$ jeweils —CO—O—, —O—CO—, —$CH_2CH_2$—, —$OCH_2$—, —$CH_2O$— oder eine Einfachbindung,

und

$R^3$ H, eine Alkylgruppe mit 1-10 C-Atomen, worin auch eine oder zwei nicht benachbarte $CH_2$-Gruppen durch O-Atome und/oder —CH=CH-Gruppen ersetzt sein können, F, Cl, Br oder CN bedeutet.

Der Einfachheit halber bedeuten im folgenden Ph eine 1,4-Phenylengruppe, die auch gegebenenfalls durch ein oder zwei F- und/oder Cl-Atome und/oder $CH_3$-Gruppen und/oder CN-Gruppen substituiert sein kann, Cy eine 1,4-Cyclohexylengruppe und Bi eine Bicyclo [2, 2, 2] octylengruppe.

Ähnliche Verbindungen sind z. B. aus US 4, 298, 528 ; GB-A-2 073 175 ; Mol. Cryst. Liq. Cryst., 1985, Vol. 123, pp. 137-14 ; GB-A-2 044 767 und GB-A-2 046 742 bekannt. Die dort angegebenen Verbindungen enthalten jedoch im Gegensatz zu den vorliegenden keine trisubstituierten Ringstrukturen.

Die Verbindungen der Formel I können wie ähnliche Verbindungen als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen, dem 2-Frequenz-Verfahren oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte, sehr kleiner optischer Anisotropie und vergleichsweise niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind ; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums wesentlich zu beeinflussen. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil. Es handelt sich um polare Substanzen, in denen hoch polare Zusätze wie Leitsalze und dichroitische Farbstoffe gut löslich sind.

Gegenstand der Erfindung sind somit die Verbindungen der Formel I sowie ein Verfahren zur Herstellung von Verbindungen der Formel I nach Anspruch 1, dadurch gekennzeichnet, daß man einen entsprechenden Aldehyd bzw. ein entsprechendes Keton mit einem entsprechenden Diol bzw. Dithiol umsetzt,
oder daß man zur Herstellung von Estern der Formel I (worin $Z^1$ und/oder $Z^2$ —CO—O— oder —O—CO— bedeuten und/oder $R^1$ und/oder $R^2$ eine Alkylgruppe bedeutet, worin eine $CH_2$-Gruppe durch —O—CO— oder —CO—O— ersetzt ist), eine entsprechende Carbonsäure oder eines ihrer reaktionsfähigen Derivate mit einem entsprechenden Alkohol oder einem seiner reaktionsfähigen Derivate umsetzt, oder daß man zur Herstellung von Nitrilen der Formel I (worin $R^1$ und/oder $R^2$ und/oder $R^3$ CN bedeutet und/oder A eine in 5-Stellung durch CN substituierte 1,3-Dioxan-2,5-diyl- oder 1,3-Dithian-2,5-diylgruppe ist) ein entsprechendes Carbonsäureamid dehydratisiert oder ein entsprechendes Carbonsäurehalogenid mit Sulfamid umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, $R^2$, $R^3$, $A^1$, $A^2$, $A^3$, $A^4$, A, $Z^1$ und $Z^2$ die angegebene Bedeutung, sofern nicht ausdrücklich etwas anderes vermerkt ist.

Die Verbindungen der Formel I umfassen dementsprechend insbesondere Verbindungen der Teilformeln Ia und Ib (mit zwei Ringen)

| | |
|---|---|
| $R^1$-A-$A^2$-$R^2$ | Ia |
| $R^1$-A-$Z^1$-$A^2$-$R^2$ | Ib |

Ic bis Ii (mit drei Ringen),

| | |
|---|---|
| $R^1$-$A^4$-A-$A^2$-$R^2$ | Ic |
| $R^1$-A-$A^4$-$A^2$-$R^2$ | Id |
| $R^1$-$A^4$-A-$Z^1$-$A^2$-$R^2$ | Ie |
| $R^1$-A-$A^4$-$Z^1$-$A^2$-$R^2$ | If |
| $R^1$-A-$Z^1$-$A^2$-$A^3$-$R^3$ | Ig |
| $R^3$-$A^3$-$Z^2$-A-$Z^1$-$A^2$-$R^2$ | Ih |
| $R^1$-A-$Z^1$-$A^2$-$Z^2$-$A^3$-$R^3$ | Ii |

sowie Ij bis It (mit vier Ringen)

| | |
|---|---|
| $R^1$-$A^4$-A-$A^2$-$A^3$-$R^3$ | Ij |
| $R^1$-A-$A^4$-$A^2$-$A^3$-$R^3$ | Ik |
| $R^3$-$A^3$-$Z^2$-$A^4$-A-$A^2$-$R^2$ | Il |
| $R^3$-$A^3$-$A^4$-A-$Z^1$-$A^2$-$R^2$ | Im |
| $R^1$-A-$A^4$-$A^2$-$Z^2$-$A^3$-$R^3$ | In |
| $R^1$-A-$A^4$-$Z^1$-$A^2$-$A^3$-$R^3$ | Io |
| $R^1$-$A^4$-A-$Z^1$-$A^2$-$A^3$-$R^3$ | Ip |
| $R^1$-$A^4$-A-$Z^1$-$A^2$-$Z^2$-$A^3$-$R^3$ | Iq |
| $R^1$-A-$A^4$-$Z^1$-$A^2$-$Z^2$-$A^3$-$R^3$ | Ir |
| $R^3$-$A^3$-$Z^2$-$A^4$-A-$Z^1$-$A^2$-$R^2$ | Is |
| $R^3$-$A^3$-$Z^2$-A-$A^4$-$Z^1$-$A^2$-$R^2$ | It |

Darunter sind diejenigen der Formeln Ia, Ib, Ic, Id, Ie, If, Ig, Ij und Ik besonders bevorzugt.
Die bevorzugten Verbindungen der Formel Ia umfassen solche der Teilformeln Ia1 bis Ia3 :

| | |
|---|---|
| $R^1$-A-Ph-$R^2$ | Ia1 |
| $R^1$-A-Cy-$R^2$ | Ia2 |
| $R^1$-A-Bi-$R^2$ | Ia3 |

Darunter sind diejenigen der Teilformeln Ia1 und Ia2 besonders bevorzugt.
Die bevorzugten Verbindungen der Formel Ib umfassen solche der Teilformeln Ib1 bis Ib3 :

| | |
|---|---|
| $R^1$-A-$Z^1$-Ph-$R^2$ | Ib1 |
| $R^1$-A-$Z^1$-Cy-$R^2$ | Ib2 |
| $R^1$-A-$Z^1$-Bi-$R^2$ | Ib3 |

Darunter sind diejenigen der Teilformeln Ib1 und Ib2, insbesondere diejenigen worin $Z^1$ —CO—O—, —O—CO— oder —$CH_2CH_2$— bedeutet, besonders bevorzugt.

Die bevorzugten Verbindungen der Formel Ic umfassen solche der Teilformeln Ic1 und Ic2 :

$R^1$-Cy-A-Cy-$R^2$ Ic1
$R^1$-Cy-A-Ph-$R^2$ Ic2

Die bevorzugten Verbindungen der Formel Id umfassen solche der Teilformeln Id1 bis Id4 :

$R^1$-A-Cy-Cy-$R^2$ Id1
$R^1$-A-Ph-Ph-$R^2$ Id2
$R^1$-A-Ph-Cy-$R^2$ Id3
$R^1$-A-Cy-Ph-$R^2$ Id4

Die bevorzugten Verbindungen der Formel Ie umfassen solche der Teilformeln Ie1 bis Ie3 :

$R^1$-Cy-A-$Z^1$-Cy-$R^2$ Ie1
$R^1$-Cy-A-$Z^1$-Ph-$R^2$ Ie2
$R^1$-Ph-A-$Z^1$-Cy-$R^2$ Ie3

Darunter sind diejenigen der Teilformel Ie1, insbesondere diejenigen worin $Z^1$ —CO—O—, —O—CO— oder —$CH_2CH_2$— bedeutet, besonders bevorzugt.

Die bevorzugten Verbindungen der Formel If umfassen solche der Teilformeln If1 bis If4 :

$R^1$-A-Cy-$Z^1$-Cy-$R^2$ If1
$R^1$-A-Ph-$Z^1$-Ph-$R^2$ If2
$R^1$-A-Ph-$Z^1$-Cy-$R^2$ If3
$R^1$-A-Cy-$Z^1$-Ph-$R^2$ If4

Darunter sind diejenigen der Teilformeln If1, If2 und If3, insbesondere diejenigen worin $Z^1$ —CO—O—, —O—CO— oder —$CH_2CH_2$—, insbesondere —CO—O—, bedeutet, besonders bevorzugt.

Die bevorzugten Verbindungen der Formel Ig umfassen solche der Teilformeln Ig1 und Ig2 :

$R^1$-A-$Z^1$-Cy-Cy-$R^3$ Ig1
$R^1$-A-$Z^1$-Ph-Cy-$R^2$ Ig2

Darunter sind diejenigen besonders bevorzugt worin $Z^1$ —O—CO—, —CO—O— oder —$CH_2CH_2$— bedeutet.

Die bevorzugten Verbindungen der Formel Ij umfassen solche der Teilformeln Ij1 und Ij2 :

$R^1$-Cy-A-Ph-Ph-$R^3$ Ij1
$R^1$-Cy-A-Ph-Cy-$R^3$ Ij2

Die bevorzugten Verbindungen der Formel Ik umfassen solche der Teilformeln Ik1 und Ik2 :

$R^1$-A-Ph-Ph-Cy-$R^3$ Ik1
$R^1$-A-Ph-Cy-Cy-$R^3$ Ik2

In den Verbindungen der vor- und nachstehenden Formeln bedeuten $R^1$, $R^2$ bzw. $R^3$ vorzugsweise Alkyl, ferner Alkoxy- oder eine andere Oxaalkylgruppe.

Weiterhin bevorzugt sind Verbindungen der vor- und nachstehenden Formeln worin einer der Reste $R^1$, $R^2$ bzw. $R^3$ —CO-Alkyl, —O—CO-Alkyl, —CO—O-Alkyl oder CN bedeutet.

In den bevorzugten Verbindungen der vor- und nachstehenden Formeln können die Alkylreste, in denen auch eine $CH_2$-Gruppe (Alkoxy bzw. Oxaalkyl) durch ein O-Atom ersetzt sein kann, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Methoxy, Octoxy oder Nonoxy.

$A^2$, $A^3$ und $A^4$ sind bevorzugt Cy oder Ph, insbesondere bevorzugt unsubstituierte 1,4-Phenylen- oder trans-1,4-Cyclohexylen-Gruppen. $Z^1$ und $Z^2$ sind bevorzugt Einfachbindungen, in zweiter Linie bevorzugt —O—CO—, —CO—O— oder $CH_2CH_2$-Gruppen.

A ist eine Gruppe ausgewählt aus den Formeln (B) und (D),

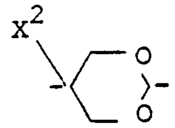

(B)

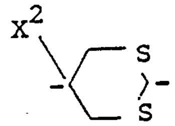

(D)

worin $X^2$ vorzugsweise CN bedeutet.

A umfaßt auch die Spiegelbilder der Formeln (B) und (D). Besonders bevorzugt sind die Gruppen (B) mit $X^2$ = CN.

Verbindungen der vor- und nachstehenden Formeln mit verzweigten Flügelgruppen $R^1$, $R^2$ bzw. $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung.

Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formel I sowie Ia bis It sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I10 :

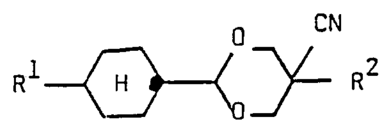

I1

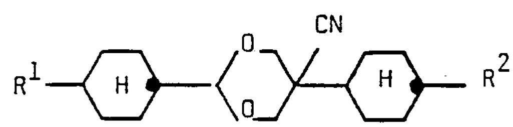

I2

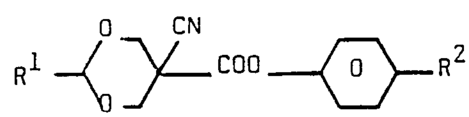

I3

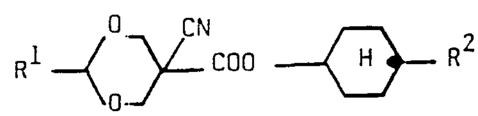

I4

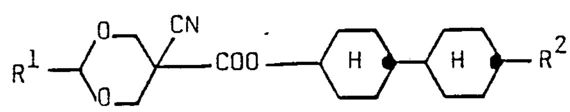

I5

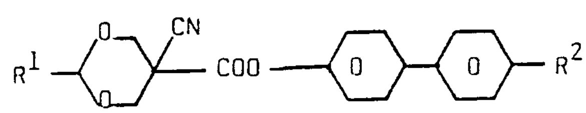

I6

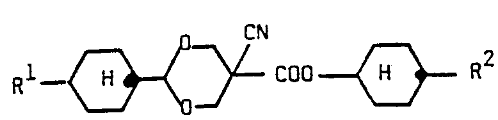

I7

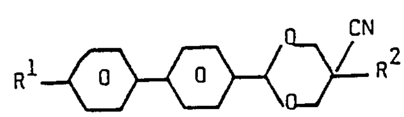

I8

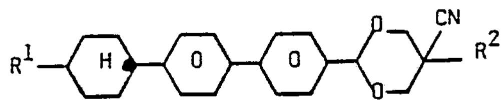

I9

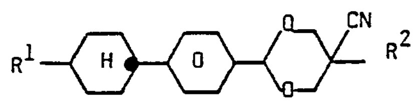

I10

In den Verbindungen der vorstehenden Formeln I1-I10 bedeuten $R^1$ und $R^2$ vorzugsweise verzweigtes oder geradkettiges Alkyl oder Alkoxy mit jeweils 2 bis 10 C-Atomen. 1,4-Phenylen-Gruppen können auch lateral durch Fluor substituiert sein.

In den Verbindungen der vorstehenden genannten Formeln sind diejenigen Stereoisomeren bevorzugt, in den die Substituenten $R^1$-, $R^1$-$A^4$-, $R^2$-$A^2$-$Z^1$- bzw. $R^2$-$A^2$-$Z^1$-$A^4$- in 2- und 5-Position des Ringes A trans-ständig sind und die äquatoriale Stellung einnehmen, während der zusätzliche Substituent an A in 5-Position ($X^2$) eine axiale Stellung einnimmt. Diese sind in der Regel stabiler; in vielen Fällen lassen sich die cis-Verbindungen (oder Gemische) durch Behandeln mit einer Base, z. B. mit K-tert.-Butylat in einem inerten Lösungsmittel wie Dimethylsulfoxid, in die trans-Verbindungen umwandeln.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, in dem man einen entsprechenden Aldehyd bzw. ein entsprechendes Keton mit einem entsprechenden Diol bzw. Dithiol umsetzt. Nachfolgend wird auf die Herstellung von Verbindungen der Formel I, worin $A^1$-A- bedeutet, näher eingegangen. Verbindungen der Formel I, worin $A^1$-$A^4$-A- oder -A-$A^4$- bedeutet, sind nach analogen Verfahren herstellbar.

In den nachstehenden Formeln (e)-(h) haben $R^1$, $R^2$, $A^2$ und $Z^1$ die oben angegebene Bedeutung. Q bedeutet O oder S. $X^2$ bedeutet F, Cl oder CN.

Verbindungen, worin A eine in 5-Stellung durch F, Cl oder CN substituierte 1,3-Dioxan-2,5-diyl- oder 1,3-Dithian-2,5-diyl-Gruppe bedeutet, sind durch Kondensation eines Aldehyds der Formel (e) mit einem Diol bzw. Dithiol der Formel (f) oder eines Ketons der Formel (g) mit einem Diol bzw. Dithiol der Formel (h) erhältlich.

$$R^1\text{-CHO} \qquad (e)$$

$$(HQ\,CH_2)_2\,CX^2\text{-}Z^1\text{-}A^2\text{-}R^2 \qquad (f)$$

$$OHC\text{-}Z^1\text{-}A^2\text{-}R^2 \qquad (g)$$

$$R^1\text{-}CX^2(CH_2QH)_2 \qquad (h)$$

Die Ausgangsmaterialien, insbesondere (e) bis (h) können als solche oder auch in Form ihrer reaktionsfähigen Derivate eingesetzt werden. Als reaktionsfähige Derivate eignen sich in erster Linie Acetale.

Die Kondensation der oben genannten Ausgangsmaterialien erfolgt vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20° und etwa 150°, vorzugsweise zwischen 80° und 120°.

Die genannten Ausgangsmaterialien sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate erhältlich. Die Ketone sind beispielsweise durch Umsetzung entsprechender Nitrile (aus den entsprechenden Carbonsäuren erhältlich) mit Grignard-Verbindungen erhältlich. Die Diole sind beispielsweise durch Reduktion entsprechender Diester erhältlich, die ihrerseits nach Standardverfahren aus Malonester hergestellt werden können. Die Dithiole sind durch Umsetzung entsprechender Dihalogenide (erhältlich aus den Diolen) mit NaSH erhältlich.

Ester der Formel I (worin $Z^1$ und/oder $Z^2$ —CO—O— oder —O—CO— bedeuten und/oder $R^1$ und/oder $R^2$ eine Alkylgruppe bedeutet, worin eine $CH_2$-Gruppe durch —O—CO— oder —CO—O— ersetzt ist) können auch durch Veresterung entsprechender Carbonsäuren mit Alkoholen bzw. Phenolen oder ihren reaktionsfähigen Derivate erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate in Betracht, vorzugsweise eines Alkalimetalls wie Na oder K.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoff wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen − 50° und + 250°, vorzugsweise zwischen − 20° und + 80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa − 25° und + 20°.

Zur Herstellung von Nitrilen der Formel I (worin $R^1$ und/oder $R^2$ und/oder $R^3$ CN bedeuten und/oder A eine in 5-Stellung durch CN substituierte 1,3-Dioxan-2,5-diyl- oder 1,3-Dithian-2,5-diyl-Gruppe ist) können entsprechende Säureamide, z. B. solche, in denen an Stelle des Restes CN eine $CONH_2$-Gruppe steht, dehydratisiert werden.

Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten ; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Die erfindungsgemäßen Dielektrika bestehen aus 2 bis 20 vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbon-säurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4′-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyldithiane, Cyclohexylethylbenzole, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel II charakterisieren,

$$R'\text{-}L\text{-}G\text{-}E\text{-}R'' \qquad II$$

worin L und E je ein carbo- oder heterocyclisches Ringsystem aus der aus 1,4-disubstituierten Benzol- und Cyclohexanringen, 4,4′-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und

Tetrahydronaphthalin, Chinazolin und Tetrahydrochinazolin gebildeten Gruppe, wobei die 1,4-disubstituierten Cyclohexanringe zusätzlich in 1- oder 4-Stellung eine Cyanogruppe tragen können,

G

| | |
|---|---|
| —CH═CH— | —N(O)═N— |
| —CH═CY— | —CH═N(O)— |
| —C≡C— | $—CH_2—CH_2—$ |
| —CO—O— | $—CH_2—O—$ |
| —CO—S— | $—CH_2—S—$ |
| —CH═N— | —COO—Phe—COO— |

oder eine C—C-Einfachbindung, Y Halogen, vorzugsweise Chlor, oder —CN, und R′ und R″ Alkyl, Alkoxy-Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R′ und R″ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Dielektrika enthalten in der Regel mindestens 30, vorzugsweise 50-99, insbesondere 60-98 Gewichtzprozent der Verbindungen der Formel I und II. Hiervon entfallen bevorzugt mindestens 5 Gewichtsprozent meist auch 10-30 Gewichtsprozent auf eine oder mehrere Verbindungen der Formel I. Jedoch werden von der Erfindung auch solche flüssigkristallinen Dielektrika umfaßt, denen beispeislweise zu Dotierungszwecken nur weniger als 5 Gewichtsprozent zum Beispiel 0,1 bis 3 Gewichtsprozent einer oder mehrerer Verbindungen der Formel I zugesetzt worden sind. Andererseits können die Verbindungen der Formel I bis zu 60 Gewichtsprozent der erfindungsgemäßen Dielektrika ausmachen. Vorzugsweise enthalten die flüssigkristallinen Dielektrika nach der Erfindung 10 bis 30 Gewichtsprozent einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur. Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. « Übliche Aufarbeitung » bedeutet: man gibt Wasser hinzu, extrahiert mit Methylenchlorid, trennt ab, trocknet die organische Phase, dampft ein und reinigt das Produkt durch Kristallisation und/oder Chromatographie.

## Beispiel 1

Man kondensiert trans, trans-4-n-Propylbicyclohexylcarbaldehyd-4′ mit α,α-Bishydroxymethylcapronsäure und überführt die erhaltene Säure durch Erhitzen mit Thionylchlorid in das Säurechlorid und anschließend durch Behandeln mit Ammoniak in das Amid. 7,9 g des Amids werden mit 36 g Thionylchlorid und 0,2 g Dimethylformamid in 200 ml Toluol 15 Stunden auf 80° erhitzt.

Anschließend wird das überschüssige Thionylchlorid und die Hälfte des Toluols abdestilliert. Die verbleibende Lösung wird mit Natriumbikarbonat und Wasser neutral gewaschen und über Natriumsulfat getrocknet. Nach dem Abdampfen des Lösungsmittels erhält man durch mehrfaches Umkristallisieren reines trans, trans-4-n-Propyl-4′-(trans-5-n-butyl-cis-5-cyano-1,3-dioxan-2-yl)-bicyclohexyl.

Analog werden hergestellt:

trans, trans-4-Propyl-4′-(trans-5-methyl-cis-5-cyano-1,3-dioxan-2-yl)-bicyclohexyl
trans, trans-4-Propyl-4′-(trans-5-ethyl-cis-5-cyano-1,3-dioxan-2-yl)-bicyclohexyl
trans, trans-4-Propyl-4′-(trans-5-propyl-cis-5-cyano-1,3-dioxan-2-yl)-bicyclohexyl
trans, trans-4-Propyl-4′-(trans-5-butyl-cis-5-cyano-1,3-dioxan-2-yl)-bicyclohexyl
trans, trans-4-Propyl-4′-(trans-5-pentyl-cis-5-cyano-1,3-dioxan-2-yl)-bicyclohexyl
trans, trans-4-Propyl-4′-(trans-5-heptyl-cis-5-cyano-1,3-dioxan-2-yl)-bicyclohexyl

trans, trans-4-Pentyl-4'-(trans-5-methyl-cis-5-cyano-1,3-dioxan-2-yl)-bicyclohexyl
trans, trans-4-Pentyl-4'-(trans-5-ethyl-cis-5-cyano-1,3-dioxan-2-yl)-bicyclohexyl
trans, trans-4-Pentyl-4'-(trans-5-propyl-cis-5-cyano-1,3-dioxan-2-yl)-bicyclohexyl
trans, trans-4-Pentyl-4'-(trans-5-butyl-cis-5-cyano-1,3-dioxan-2-yl)-bicyclohexyl
trans, trans-4-Pentyl-4'-(trans-5-pentyl-cis-5-cyano-1,3-dioxan-2-yl)-bicyclohexyl
trans, trans-4-Pentyl-4'-(trans-5-heptyl-cis-5-cyano-1,3-dioxan-2-yl)-bicyclohexyl

trans-4-Propyl-(trans-5-methyl-cis-5-cyano-1,3-dioxan-2-yl)-cyclohexan
trans-4-Propyl-(trans-5-ethyl-cis-5-cyano-1,3-dioxan-2-yl)-cyclohexan
trans-4-Propyl-(trans-5-propyl-cis-5-cyano-1,3-dioxan-2-yl)-cyclohexan
trans-4-Propyl-(trans-5-butyl-cis-5-cyano-1,3-dioxan-2-yl)-cyclohexan
trans-4-Propyl-(trans-5-pentyl-cis-5-cyano-1,3-dioxan-2-yl)-cyclohexan
trans-4-Propyl-(trans-5-heptyl-cis-5-cyano-1,3-dioxan-2-yl)-cyclohexan

trans-4-Pentyl-(trans-5-methyl-cis-5-cyano-1,3-dioxan-2-yl)-cyclohexan
trans-4-Pentyl-(trans-5-ethyl-cis-5-cyano-1,3-dioxan-2-yl)-cyclohexan
trans-4-Pentyl-(trans-5-propyl-cis-5-cyano-1,3-dioxan-2-yl)-cyclohexan
trans-4-Pentyl-(trans-5-butyl-cis-5-cyano-1,3-dioxan-2-yl)-cyclohexan
trans-4-Pentyl-(trans-5-mentyl-cis-5-cyano-1,3-dioxan-2-yl)-cyclohexan
trans-4-Pentyl-(trans-5-heptyl-cis-5-cyano-1,3-dioxan-2-yl)-cyclohexan

trans-4-Butyl-(trans-5-methyl-cis-5-cyano-1,3-dioxan-2-yl)-cyclohexan
trans-4-Butyl-(trans-5-ethyl-cis-5-cyano-1,3-dioxan-2-yl)-cyclohexan
trans-4-Butyl-(trans-5-propyl-cis-5-cyano-1,3-dioxan-2-yl)-cyclohexan
trans-4-Butyl-(trans-5-butyl-cis-5-cyano-1,3-dioxan-2-yl)-cyclohexan
trans-4-Butyl-(trans-5-pentyl-cis-5-cyano-1,3-dioxan-2-yl)-cyclohexan
trans-4-Butyl-(trans-5-heptyl-cis-5-cyano-1,3-dioxan-2-yl)-cyclohexan

## Beispiel 2

Man löst 24,8 g 2-n-Pentyl-5-cyan-1,3-dioxan-5-carbonsäurechlorid [erhältlich durch Umsetzung von Cyanessigester mit Formaldehyd in Gegenwert von Natriumhydrogencarbonat, nachfolgender säurekatalysierter Kondensation mit Hexanal, Verseifung des erhaltenen 2-n-Pentyl-5-cyan-1,3-dioxan-5-carbonsäureethylesters mit 10 %iger wäßrig-alkoholischer KOH und Überführung der freien Säure mit Cyanurchlorid/Triethylamin in das Säurechlorid] in 150 ml Toluol, versetzt mit 8 ml Pyridin und 14 g trans-4-n-Propylcyclohexanol und kocht zwei Stunden. Nach Abkühlen, üblicher Aufarbeitung und Isomerentrennung über die Thioharnstoff-Einschlußverbindung erhält man 2-n-Pentyl-cis-5-cyan-1,3-dioxan-trans-5-carbonsäure-(trans-4-n-propylcyclohexylester), F. 107°.

Analog werden hergestellt :

2-Pentyl-cis-5-cyan-1,3-dioxan-trans-5-carbonsäure-(trans-4-ethylcyclohexylester)
2-Pentyl-cis-5-cyan-1,3-dioxan-trans-5-carbonsäure-(trans-4-butylcyclohexylester)
2-Pentyl-cis-5-cyan-1,3-dioxan-trans-5-carbonsäure-(trans-4-pentylcyclohexylester), F. 111°, K. 91°
2-Pentyl-cis-5-cyan-1,3-dioxan-trans-5-carbonsäure-(trans-4-heptylcyclohexylester)

2-Butyl-cis-5-cyan-1,3-dioxan-trans-5-carbonsäure-(trans-4-ethylcyclohexylester)
2-Butyl-cis-5-cyan-1,3-dioxan-trans-5-carbonsäure-(trans-4-propylcyclohexylester)
2-Butyl-cis-5-cyan-1,3-dioxan-trans-5-carbonsäure-(trans-4-butylcyclohexylester)
2-Butyl-cis-5-cyan-1,3-dioxan-trans-5-carbonsäure-(trans-4-pentylcyclohexylester)
2-Butyl-cis-5-cyan-1,3-dioxan-trans-5-carbonsäure-(trans-4-heptylcyclohexylester)

2-Propyl-cis-5-cyan-1,3-dioxan-trans-5-carbonsäure-(trans-4-ethylcyclohexylester)
2-Propyl-cis-5-cyan-1,3-dioxan-trans-5-carbonsäure-(trans-4-propylcyclohexylester)
2-Propyl-cis-5-cyan-1,3-dioxan-trans-5-carbonsäure-(trans-4-butylcyclohexylester)
2-Propyl-cis-5-cyan-1,3-dioxan-trans-5-carbonsäure-(trans-4-pentylcyclohexylester)
2-Propyl-cis-5-cyan-1,3-dioxan-trans-5-carbonsäure-(trans-4-heptylcyclohexylester)

2-Propyl-cis-5-cyan-1,3-dioxan-trans-5-carbonsäure-(p-methylphenylester)
2-Propyl-cis-5-cyan-1,3-dioxan-trans-5-carbonsäure-(p-ethylphenylester)
2-Propyl-cis-5-cyan-1,3-dioxan-trans-5-carbonsäure-(p-propylphenylester)
2-Propyl-cis-5-cyan-1,3-dioxan-trans-5-carbonsäure-(p-butylphenylester)
2-Propyl-cis-5-cyan-1,3-dioxan-trans-5-carbonsäure-(p-pentylphenylester)
2-Propyl-cis-5-cyan-1,3-dioxan-trans-5-carbonsäure-(p-heptylphenylester)
2-Propyl-cis-5-cyan-1,3-dioxan-trans-5-carbonsäure-(p-methoxyphenylester)

2-Propyl-cis-5-cyan-1,3-dioxan-trans-5-carbonsäure-(p-ethoxyphenylester)
2-Propyl-cis-5-cyan-1,3-dioxan-trans-5-carbonsäure-(p-propoxyphenylester)
2-Propyl-cis-5-cyan-1,3-dioxan-trans-5-carbonsäure-(p-butoxyphenylester)
2-Propyl-cis-5-cyan-1,3-dioxan-trans-5-carbonsäure-(p-cyanphenylester)

2-Butyl-cis-5-cyan-1,3-dioxan-trans-5-carbonsäure-(p-methylphenylester)
2-Butyl-cis-5-cyan-1,3-dioxan-trans-5-carbonsäure-(p-ethylphenylester)
2-Butyl-cis-5-cyan-1,3-dioxan-trans-5-carbonsäure-(p-propylphenylester)
2-Butyl-cis-5-cyan-1,3-dioxan-trans-5-carbonsäure-(p-butylphenylester)
2-Butyl-cis-5-cyan-1,3-dioxan-trans-5-carbonsäure-(p-pentylphenylester)
2-Butyl-cis-5-cyan-1,3-dioxan-trans-5-carbonsäure-(p-heptylphenylester)
2-Butyl-cis-5-cyan-1,3-dioxan-trans-5-carbonsäure-(p-methoxyphenylester)
2-Butyl-cis-5-cyan-1,3-dioxan-trans-5-carbonsäure-(p-ethoxyphenylester)
2-Butyl-cis-5-cyan-1,3-dioxan-trans-5-carbonsäure-(p-propoxyphenylester)
2-Butyl-cis-5-cyan-1,3-dioxan-trans-5-carbonsäure-(p-butoxyphenylester)
2-Butyl-cis-5-cyan-1,3-dioxan-trans-5-carbonsäure-(p-cyanphenylester)

2-Pentyl-cis-5-cyan-1,3-dioxan-trans-5-carbonsäure-(p-methylphenylester)
2-Pentyl-cis-5-cyan-1,3-dioxan-trans-5-carbonsäure-(p-ethylphenylester)
2-Pentyl-cis-5-cyan-1,3-dioxan-trans-5-carbonsäure-(p-propylphenylester), F. 63°
2-Pentyl-cis-5-cyan-1,3-dioxan-trans-5-carbonsäure-(p-butylphenylester)
2-Pentyl-cis-5-cyan-1,3-dioxan-trans-5-carbonsäure-(p-pentylphenylester)
2-Pentyl-cis-5-cyan-1,3-dioxan-trans-5-carbonsäure-(p-heptylphenylester)

2-Pentyl-cis-5-cyan-1,3-dioxan-trans-5-carbonsäure-(p-methoxyphenylester), F. 68°
2-Pentyl-cis-5-cyan-1,3-dioxan-trans-5-carbonsäure-(p-ethoxyphenylester)
2-Pentyl-cis-5-cyan-1,3-dioxan-trans-5-carbonsäure-(p-propoxyphenylester)
2-Pentyl-cis-5-cyan-1,3-dioxan-trans-5-carbonsäure-(p-butoxyphenylester)
2-Pentyl-cis-5-cyan-1,3-dioxan-trans-5-carbonsäure-(p-cyanphenylester)

2-Propyl-cis-5-cyan-1,3-dioxan-trans-5-carbonsäure-(trans, trans-4'-methylbicyclohex-4-ylester)
2-Propyl-cis-5-cyan-1,3-dioxan-trans-5-carbonsäure-(trans, trans-4'-ethylbicyclohex-4-ylester)
2-Propyl-cis-5-cyan-1,3-dioxan-trans-5-carbonsäure-(trans, trans-4'-propylbicyclohex-4-ylester)
2-Propyl-cis-5-cyan-1,3-dioxan-trans-5-carbonsäure-(trans, trans-4'-butylbicyclohex-4-ylester)
2-Propyl-cis-5-cyan-1,3-dioxan-trans-5-carbonsäure-(trans, trans-4'-pentylbicyclohex-4-ylester)
2-Propyl-cis-5-cyan-1,3-dioxan-trans-5-carbonsäure-(trans, trans-4'-heptylbicyclohex-4-ylester)

2-Pentyl-cis-5-cyan-1,3-dioxan-trans-5-carbonsäure-(trans, trans-4'-methylbicyclohex-4-ylester)
2-Pentyl-cis-5-cyan-1,3-dioxan-trans-5-carbonsäure-(trans, trans-4'-ethylbicyclohex-4-ylester)
2-Pentyl-cis-5-cyan-1,3-dioxan-trans-5-carbonsäure-(trans, trans-4'-propylbicyclohex-4-ylester)
2-Pentyl-cis-5-cyan-1,3-dioxan-trans-5-carbonsäure-(trans, trans-4'-butylbicyclohex-4-ylester)
2-Pentyl-cis-5-cyan-1,3-dioxan-trans-5-carbonsäure-(trans, trans-4'-pentylbicyclohex-4-ylester)
2-Pentyl-cis-5-cyan-1,3-dioxan-trans-5-carbonsäure-(trans, trans-4'-heptylbicyclohex-4-ylester)

2-(trans-4-Propylcyclohexyl)-cis-5-cyan-1,3-dioxan-trans-5-carbonsäure-(trans-4-methylcyclohexylester)
2-(trans-4-Propylcyclohexyl)-cis-5-cyan-1,3-dioxan-trans-5-carbonsäure-(trans-4-ethylcyclohexylester)
2-(trans-4-Propylcyclohexyl)-cis-5-cyan-1,3-dioxan-trans-5-carbonsäure-(trans-4-propylcyclohexylester)
2-(trans-4-Propylcyclohexyl)-cis-5-cyan-1,3-dioxan-trans-5-carbonsäure-(trans-4-butylcyclohexylester)
2-(trans-4-Propylcyclohexyl)-cis-5-cyan-1,3-dioxan-trans-5-carbonsäure-(trans-4-pentylcyclohexylester)
2-(trans-4-Propylcyclohexyl)-cis-5-cyan-1,3-dioxan-trans-5-carbonsäure-(trans-4-heptylcyclohexylester)

2-(trans-4-Butylcyclohexvl)-cis-5-cyan-1,3-dioxan-trans-5-carbonsäure-(trans-4-methylcyclohexylester)
2-(trans-4-Butylcyclohexyl)-cis-5-cyan-1,3-dioxan-trans-5-carbonsäure-(trans-4-ethylcyclohexylester)
2-(trans-4-Butylcyclohexyl)-cis-5-cyan-1,3-dioxan-trans-5-carbonsäure-(trans-4-propylcyclohexylester)
2-(trans-4-Butylcyclohexyl)-cis-5-cyan-1,3-dioxan-trans-5-carbonsäure-(trans-4-butylcyclohexylester)
2-(trans-4-Butylcyclohexyl)-cis-5-cyan-1,3-dioxan-trans-5-carbonsäure-(trans-4-pentylcyclohexylester)

2-(trans-4-Butylcyclohexyl)-cis-5-cyan-1,3-dioxan-trans-5-carbonsäure-(trans-4-heptylcyclohexylester)

2-(trans-4-Pentylcyclohexyl)-cis-5-cyan-1,3-dioxan-trans-5-carbonsäure-(trans-4-methylcyclohexylester)
2-(trans-4-Pentylcyclohexyl)-cis-5-cyan-1,3-dioxan-trans-5-carbonsäure-(trans-4-ethylcyclohexylester)
2-(trans-4-Pentylcyclohexyl)-cis-5-cyan-1,3-dioxan-trans-5-carbonsäure-(trans-4-propylcyclohexylester)
2-(trans-4-Pentylcyclohexyl)-cis-5-cyan-1,3-dioxan-trans-5-carbonsäure-(trans-4-butylcyclohexylester)
2-(trans-4-Pentylcyclohexyl)-cis-5-cyan-1,3-dioxan-trans-5-carbonsäure-(trans-4-pentylcyclohexylester)
2-(trans-4-Pentylcyclohexyl)-cis-5-cyan-1,3-dioxan-trans-5-carbonsäure-(trans-4-heptylcyclohexylester)

Die folgenden Beispiele betreffen erfindungsgemäße flüssigkristalline Phasen.

Beispiel A

Man stellt eine flüssigkristalline Phase her bestehend aus

35,0 % r-1-Cyan-cis-4-(trans-4-butylcyclohexyl)-1-heptylcyclohexan,
30,0 % trans-4-Pentyl-(trans-5-pentyl-cis-5-cyano-1,3-dioxan-2-yl)-cyclohexan,
10,8 % trans, trans-4-Propyl-4′-methoxycyclohexylcyclohexan,
9,7 % trans, trans-4-Propyl-4′-ethoxycyclohexylcyclohexan,
3,9 % trans, trans-4-Butylcyclohexylcyclohexan-4′-carbonsäure-trans-4-propylcyclohexylester,
3,9 % trans, trans-4-Butylcyclohexylcyclohexan-4′-carbonsäure-trans-4-pentylcyclohexylester,
4,8 % trans, trans-4-Propylcyclohexylcyclohexan-4′-carbonsäure-trans-4-propylcyclohexylester und
3,9 % r-1-Cyan-cis-4-(trans-4-pentylcyclohexyl)-1-(trans-4-propylcyclohexyl)-cyclohexan.

## Patentansprüche

1. Heterocyclische Verbindungen der Formel I

$$R^1\text{-}A^1\text{-}Z^1\text{-}A^2\text{-}R^2 \qquad I$$

worin
$R^1$ und $R^2$ jeweils eine Alkylgruppe mit 1-10 C-Atomen, worin auch eine oder zwei nicht benachbarte $CH_2$-Gruppen durch O-Atome und/oder CO-Gruppen und/oder —OCO-Gruppen und/oder —COO-Gruppen und/oder —CH=CH-Gruppen ersetzt sein können, F, Cl, Br, CN oder $R^3$-$A^3$-$Z^2$-,
$A^1$ -A-, -$A^4$-A- oder -A-$A^4$-,
A eine Gruppe ausgewählt aus den Formeln (B) und (D)

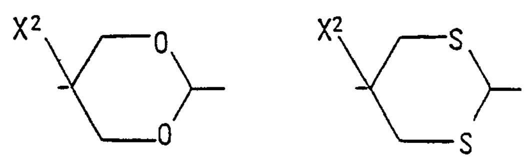

(B) (D)

worin $X^2$ F, Cl oder CN bedeutet;
$A^2$, $A^3$ und $A^4$ jeweils unsubstituierte oder durch ein oder zwei F- und/oder Cl-Atome und/oder $CH_3$-Gruppen und/oder CN-Gruppen substituierte 1,4-Phenylen-Gruppen, worin auch eine oder zwei CH-Gruppen durch N-Atome ersetzt sein können, 1,4-Cyclohexylen-Gruppen, worin auch eine oder zwei nicht benachbarte $CH_2$-Gruppen durch O-Atome ersetzt sein können, 1,3-Dithian-2,5-diyl-, Piperidin-1,4-diyl-, 1,4-Bicyclo[2,2,2]-octylen-, Decahydronaphthalin-2,6-diyl- oder 1,2,3,4-Tetrahydronaphthalin-2,6-diyl-Gruppen,
$Z^1$ und $Z^2$ jeweils —CO—O, —O—CO—, —$CH_2CH_2$—, —$OCH_2$—, —$CH_2O$— oder eine Einfachbindung,
und
$R^3$ H, eine Alkylgruppe mit 1-10 C-Atomen, worin auch eine oder zwei nicht benachbarte $CH_2$-Gruppen durch O-Atome und/oder —CH=CH-Gruppen ersetzt sein können, F, Cl, Br oder CN bedeutet.

2. Verbindungen nach Anspruch 1, gekennzeichnet durch die Formeln I1 bis I10,

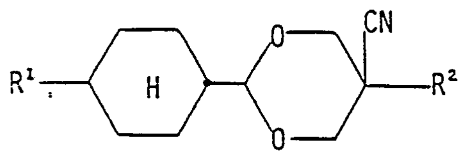

I1

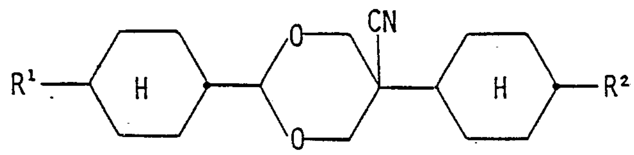

I2

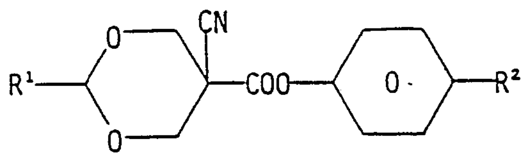

I3

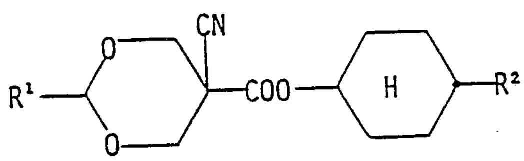

I4

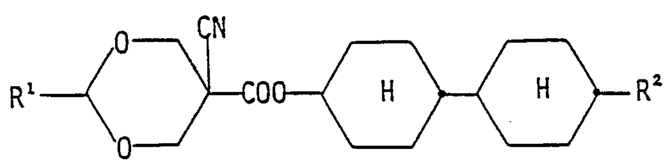

I5

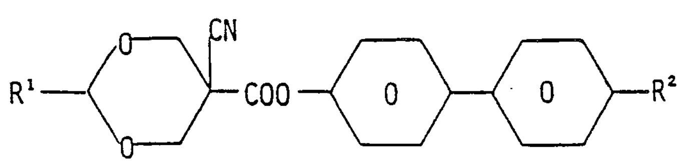

I6

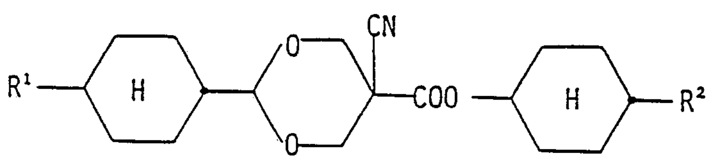

I7

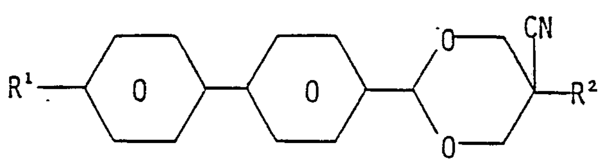

I8

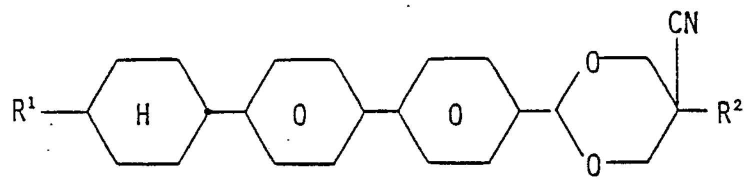

I9

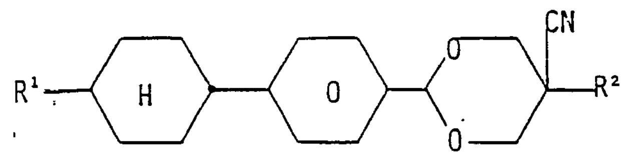

I10

worin $R^1$ und $R^2$ verzweigtes oder geradkettiges Alkyl oder Alkoxy mit jeweils 2 bis 10 C-Atomen bedeuten und die 1,4-Phenylen-Gruppen auch lateral durch Fluor substituiert sein können.

3. Verfahren zur Herstellung von Verbindungen der Formel I nach Anspruch 1, dadurch gekennzeichnet, daß man einen entsprechenden Aldehyd bzw. ein entsprechendes Keton mit einem entsprechenden Diol bzw. Dithiol umsetzt,
oder daß man zur Herstellung von Estern der Formel I (worin $Z^1$ und/oder $Z^2$ —CO—O— oder —O—CO— bedeuten und/oder $R^1$ und/oder $R^2$ eine Alkylgruppe bedeutet, worin eine $CH_2$-Gruppe durch —O—CO— oder —CO—O— ersetzt ist), eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Nitrilen der Formel I (worin $R^1$ und/oder $R^2$ und/oder $R^3$ CN bedeutet und/oder A eine in 5-Stellung durch CM substituierte 1,3-Dioxan-2,5-diyl- oder 1,3-Dithian-2,5-diylgruppe ist) ein entsprechendes Carbonsäureamid dehydratisiert oder ein entsprechendes Carbonsäurehalogenid mit Sulfamid umsetzt.

4. Verwendung der Verbindung der Formel I nach Anspruch 1 als Komponenten flüssigkristalliner Phasen.

5. Flüssigkristalline Phasen mit mindestens zwei flüssigkristallinen Komponenten, dadurch gekennzeichnet, daß mindestens eine Komponente eine Verbindung der Formel I nach Anspruch 1 ist.

6. Flüssigkristallanzeigeelement, dadurch gekennzeichnet, daß es eine Phase nach Anspruch 5 enthält.

7. Elektrooptisches Anzeigeelement, dadurch gekennzeichnet, daß es als Dielektrikum eine Phase nach Anspruch 5 enthält.

## Claims

1. Heterocyclic compounds of the formula I

$$R^1\text{-}A^1\text{-}Z^1\text{-}A^2\text{-}R^2 \qquad \text{I}$$

wherein
$R^1$ and $R^2$ are each an alkyl group having 1-10 C atoms, in which one or two non-adjacent $CH_2$ groups can also be replaced by 0 atoms and/or CO groups and/or —OCO— groups and/or —COO— groups and/or —CH═CH— groups, or are F, Cl, Br, CN or $R^3\text{-}A^3\text{-}Z^2\text{-}$,
$A^1$ is -A-, $\text{-}A^4\text{-}A\text{-}$ or $\text{-}A\text{-}A^4\text{-}$,
A is a group selected from the formulae (B) and (D)

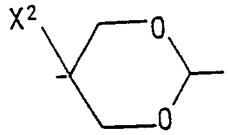

(B)

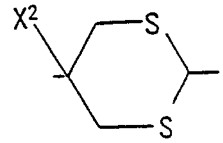

(D)

wherein $X^2$ denotes F, Cl or CN,
$A^2$, $A^3$ and $A^4$ each are 1,3-dithiane-2,5-diyl, piperidine-1,4-diyl, 1,4-bicyclo[2,2,2]octylene, decahydronaphthalene-2,6-diyl or 1,2,3,4-tetrahydronaphthalene-2,6-diyl groups, 1,4-phenylene groups, which are substituted by one or two F and/or Cl atoms and/or $CH_3$ groups and/or CN groups and in which one or two CH groups can also be replaced by N atoms, or 1,4-cyclohexylene groups in which one or two non-adjacent $CH_2$ groups can also be replaced by O atoms,

$Z^1$ and $Z^2$ each are —CO—O, —O—CO—, $—CH_2CH_2—$, $—OCH_2—$, $—CH_2O—$ or a single bond, and

$R^3$ is H, an alkyl group having 1-10 C atoms, in which one or two non-adjacent $CH_2$ groups can also be replaced by O atoms and/or —CH=CH— groups, or is F, Cl, Br, or CN.

2. Compounds of Claim 1, characterized by the formulae I1 to I10

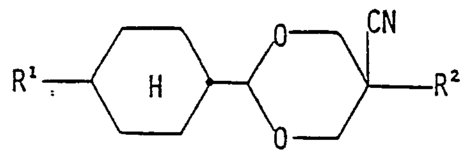

I1

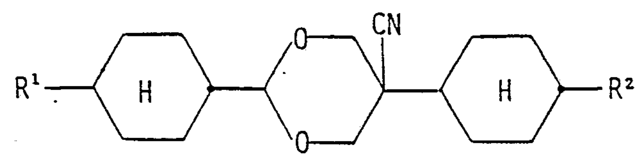

I2

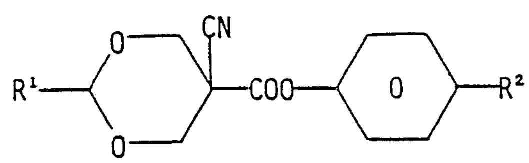

I3

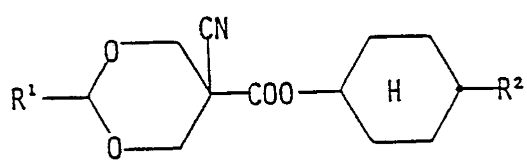

I4

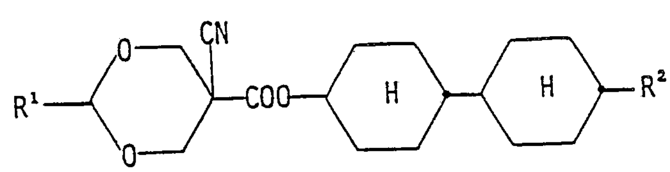

I5

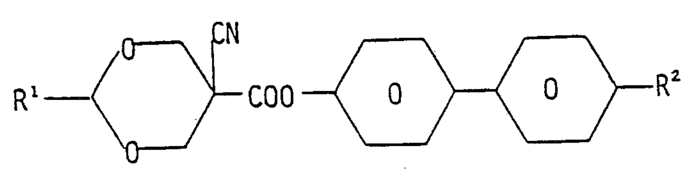

I6

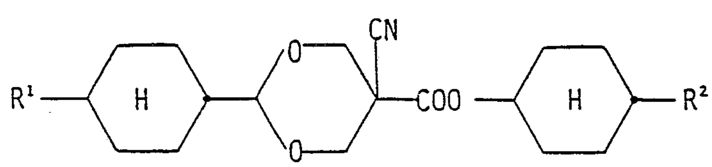

I7

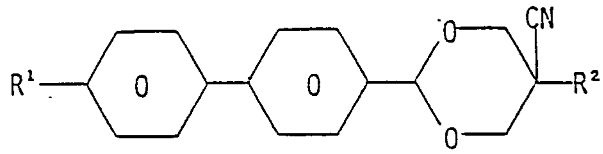

I8

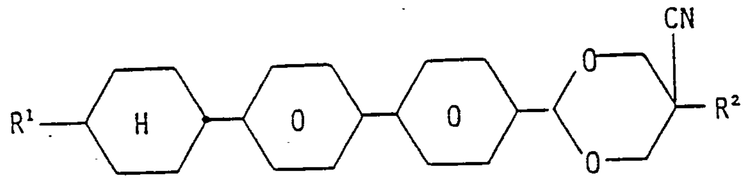

I9

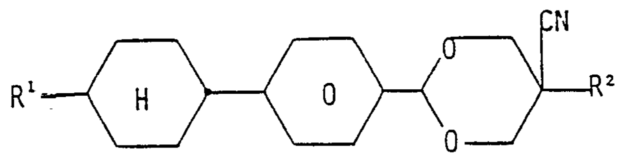

I10

wherein $R^1$ and $R^2$ denote branched or straight-chained alkyl or alkoxy with in each case 2 to 10 carbon atoms and wherein the 1,4-phenylene group may also be laterally substituted by fluorine.

3. Process for the preparation of compounds of the formula I according to Claim 1, characterised in that a corresponding aldehyde or a corresponding ketone is reacted with a corresponding diol or dithiol, or that, for the preparation of esters of the formula I (wherein $Z^1$ and/or $Z^2$ are —CO—O— or —O—CO— and/or $R^1$ and/or $R^2$ are an alkyl group in which one $CH_2$ group is replaced by —O—CO— or —CO—O—), a corresponding carboxylic acid or a reactive derivative thereof is reacted with a corresponding alcohol or a reactive derivative thereof, or that, for the preparation of nitriles of the formula I (wherein $R^1$ and/or $R^2$ and/or $R^3$ are CN and/or A is a 1,3-dioxane-2,5-diyl or 1,3-dithiane-2,5-diyl group substituted in the 5-position by CN), a corresponding carboxamide is dehydrated or a corresponding carboxylic acid halide is reacted with sulfamide.

4. Use of the compounds of the formula I according to Claim 1 as components of liquid-crystalline phases.

5. Liquid-crystalline phase with at least two liquid-crystalline components, characterised in that at least one component is a compound of the formula I according to Claim 1.

6. Liquid crystal display element, characterised in that it contains a phase according to Claim 5.

7. Electro-optical display element, characterised in that it contains a phase according to Claim 5 as the dielectric.

## Revendications

1. Composés hétérocycliques de formule I :

$$R^1\text{-}A^1\text{-}Z^1\text{-}A^2\text{-}R^2 \qquad I$$

dans laquelle :

$R^1$ et $R^2$ représentent chacun, indépendamment l'un de l'autre, un groupe alkyle en $C_1$-$C_{10}$ dans lequel un ou deux groupes $CH_2$ non voisins peuvent être remplacés par des atomes d'oxygène et/ou des groupes CO et/ou des groupes —OCO— et/ou des groupes —COO— et/ou des groupes —CH=CH—, F, Cl, Br, CN ou $R^3$-$A^3$-$Z^2$-,

$A^1$ représente -A-, -$A^4$-A- ou -A-$A^4$-,

A représente un groupe choisi parmi les groupes de formules (B) et (D) :

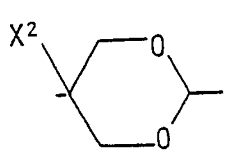

(B)

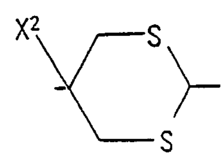

(D)

dans lesquelles $X^2$ représente F, Cl ou CN,

$A^2$, $A^3$ et $A^4$ représentent chacun un groupe 1,4-phénylène non substitué ou substitué par un ou deux atomes de F et/ou de Cl et/ou groupes $CH_3$ et/ou groupes CN et dans lequel un ou deux groupes CH peuvent être remplacés par des atomes de N, un groupe 1,4-cyclohexylène dans lequel un ou deux groupes $CH_2$ non voisins peuvent être remplacés par des atomes de O, un groupe 1,3-dithianne-2,5-diyle, pipéridine-1,4-diyle, 1,4-bicyclo[2,2,2]-octylène, décahydronaphtalène-2,6-diyle ou 1,2,3,4-tétrahydronaphtalène-2,6-diyle.

$Z^1$ et $Z^2$ représentent chacun $-CO-O$, $-O-CO$, $-CH_2CH_2-$, $-OCH_2-$, $-CH_2O-$ ou une liaison simple, et

$R^3$ représente H, un groupe alkyle en $C_1$-$C_{10}$ dans lequel un ou deux groupes $CH_2$ non voisins peuvent être remplacés par des atomes de O et/ou des groupes $-CH{=}CH-$, F, Cl, Br, ou CN.

2. Composés selon la revendication 1, caractérisés par les formules I1 à I10 :

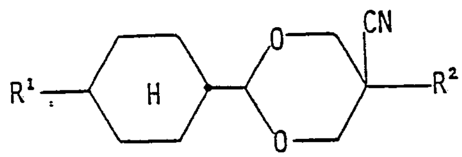

I1

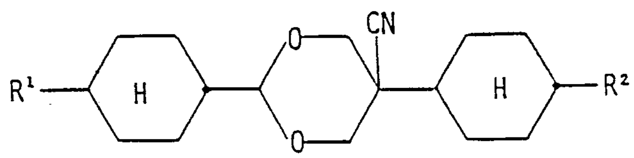

I2

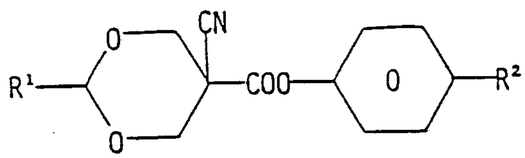

I3

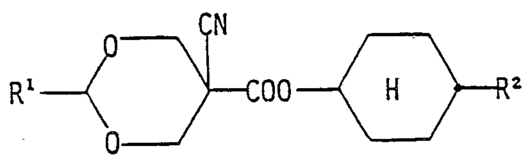

I4

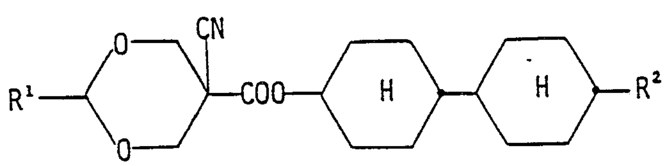

I5

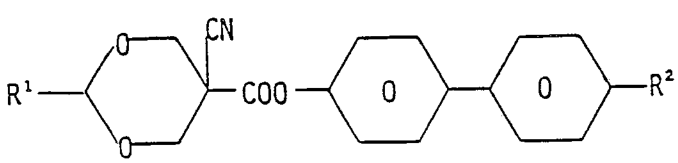

I6

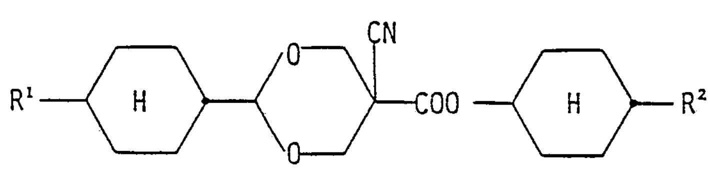

I7

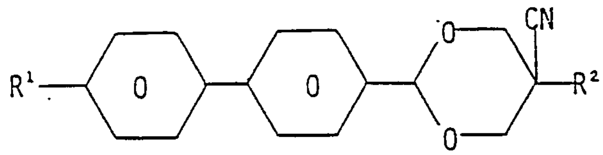

I8

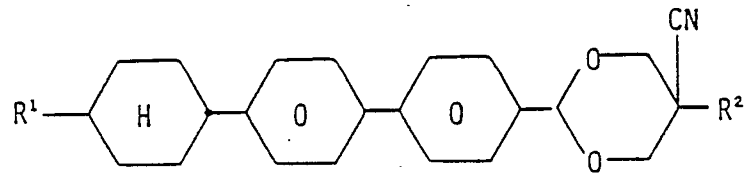

I9

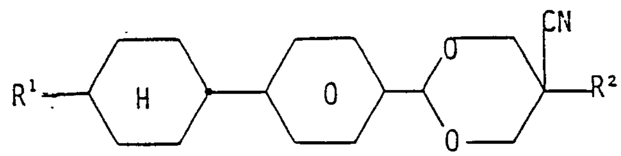

I10

dans lesquelles $R^1$ et $R^2$ représentent des groupes alkyle ou alcoxy à chaîne droite ou ramifiée contenant chacun 2 à 10 atomes de carbone, et les groupes 1,4-phénylène peuvent également être substitués en position latérale par le fluor.

3. Procédé de préparation des composés de formule I selon la revendication 1, caractérisé en ce que l'on fait réagir un aldéhyde correspondant ou une cétone correspondante respectivement avec un diol ou un dithiol correspondant, ou bien, pour la préparation des esters de formule I (dans lesquels $Z^1$ et/ou $Z^2$ représentent —CO—O— ou —O—CO— et/ou $R^1$ et/ou $R^2$ représentent des groupes alkyle dans lesquels un groupe $CH_2$ peut être remplacé par —O—CO— ou —CO—O—), on fait réagir un acide carboxylique correspondant ou l'un de ses dérivés réactifs avec un alcool correspondant ou l'un de ses dérivés réactifs, ou bien, pour la préparation des nitriles de formule I (dans lesquels $R^1$ et/ou $R^2$ et/ou $R^3$ représentent CN et/ou A représente un groupe 1,3-dioxanne-2,5-diyle ou 1,3-dithianne-2,5-diyle substitué en position 5 par CM, on déshydrate un carboxamide correspondant ou bien on fait réagir un halogénure d'acide carboxylique correspondant avec le sulfonamide.

4. Utilisation du composé de formule I selon la revendication 1 en tant que composant de phases à cristaux liquides.

5. Phases à cristaux liquides contenant au moins deux composants à cristaux liquides, caractérisées en ce que l'un au moins des composants est un composé de formule I selon la revendication 1.

6. Elément d'affichage à cristaux liquides, caractérisé en ce qu'il contient une phase selon la revendication 5.

7. Elément d'affichage électro-optique, caractérisé en ce qu'il contient en tant que diélectrique une phase selon la revendication 5.